(19) Europäisches Patentamt
European Patent Office
Office européen des brevets

(11) EP 3 925 593 A1

(12) EUROPEAN PATENT APPLICATION
published in accordance with Art. 153(4) EPC

(43) Date of publication:
22.12.2021 Bulletin 2021/51

(21) Application number: 20755452.8

(22) Date of filing: 13.02.2020

(51) Int Cl.:
*A61K 6/831* (2020.01) *A61K 6/887* (2020.01)
*A61C 5/77* (2017.01) *A61L 27/10* (2006.01)
*A61L 27/16* (2006.01) *A61L 27/44* (2006.01)
*A61L 27/50* (2006.01)

(86) International application number:
PCT/JP2020/005604

(87) International publication number:
WO 2020/166667 (20.08.2020 Gazette 2020/34)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 14.02.2019 JP 2019024811

(71) Applicant: Kuraray Noritake Dental Inc.
Kurashiki-shi, Okayama 710-0801 (JP)

(72) Inventors:
- HORIGUCHI, Hirotaka
Tainai-shi, Niigata 959-2653 (JP)
- NISHIGAKI, Naoki
Tainai-shi, Niigata 959-2653 (JP)

(74) Representative: D Young & Co LLP
120 Holborn
London EC1N 2DY (GB)

# (54) DENTAL MILL BLANK

(57) The present invention provides a dental mill blank having an excellent mechanical strength and an excellent water resistance according to which a decrease in mechanical strength can be suppressed even in an environment where water is present, for example, inside an oral cavity. The present invention relates to a dental mill blank including: a polymer (A); and continuous inorganic fibers (B) that are oriented substantially in one direction, wherein the dental mill blank has a decreasing rate of 40% or less in three-point flexural strength after immersion in 70°C water for seven days.

EP 3 925 593 A1

## Description

### TECHNICAL FIELD

The present invention relates to a dental mill blank.

### BACKGROUND ART

Recent years have seen an increasing number of application examples of a CAD/CAM system in which dental prostheses such as inlays and crowns are fabricated by designing with a computer and machining with a milling machine.

In such a system, a suitably sized block of a shape such as a rectangular parallelepiped, a cylinder, or a disc is introduced and set on a cutting machine, and milled into a restoration having a shape of crown, dentition, or the like. Various types of materials for blocks have been proposed, such as glass ceramics, zirconia, titanium, acrylic resins, and composite materials containing a polymer resin and an inorganic filling material.

Such blocks are also used for dental abutment construction materials, also called core materials. Particularly in recent years, there have been proposed mill blanks for dental abutment construction that have an improved mechanical strength owing to including continuous inorganic fibers such as glasses in a polymer resin.

For example, Patent Literature 1 proposes a dental mill blank including a sheet laminate of continuous inorganic fibers such as glasses that are cloth-like woven and a polymer resin, and also describes the use for dental abutment construction materials as one application of the dental mill blank.

Furthermore, Patent Literatures 2 and 3 propose dental mill blanks including continuous inorganic fibers such as glasses that are oriented substantially in one direction and a polymer resin, and also describe the use for dental abutment construction materials as one application of the dental mill blanks.

### CITATION LIST

#### Patent Literature

Patent Literature 1: WO 2010/109496
Patent Literature 2: WO 2017/098096
Patent Literature 3: WO 2018/195639

### SUMMARY OF INVENTION

#### Technical Problem

However, studies made by the present inventors found that the dental mill blank of Patent Literature 1 needs to be improved in terms of mechanical strength.

Also, Patent Literatures 2 and 3 are silent on the water resistance of the dental mill blanks. Studies made by the present inventors found that immersion in water for a long period significantly decreases the mechanical strength of the dental mill blanks.

In view of these, the present invention aims to provide a dental mill blank having an excellent mechanical strength and an excellent water resistance according to which a decrease in mechanical strength can be suppressed even in an environment where water is present, for example, inside an oral cavity.

#### Solution to Problem

The present invention includes the following inventions.

[1] A dental mill blank comprising:

a polymer (A); and
continuous inorganic fibers (B) that are oriented substantially in one direction, wherein
the dental mill blank has a decreasing rate of 40% or less in three-point flexural strength after immersion in 70°C water for seven days.

[2] The dental mill blank according to [1], having a water absorption amount of 23 mg/g or less after immersion in 70°C water for seven days.

[3] The dental mill blank according to [1] or [2], wherein
the polymer (A) has a water absorption amount of 30 mg/g or less after immersion in 70°C water for seven days.

[4] The dental mill blank according to any one of [1] to [3], wherein
a polymerizable monomer (a) constituting a structural unit of the polymer (A) is a radical polymerizable monomer.

[5] The dental mill blank according to any one of [1] to [4], wherein
90 mass% or more of a polymerizable monomer (a) constituting a structural unit of the polymer (A) is a polymerizable monomer (a1) having no hydroxy group.

[6] The dental mill blank according to [5], wherein
the polymerizable monomer (a1) having no hydroxy group comprises at least one selected from the group consisting of a (meth)acrylate polymerizable monomer (a1-1) having an aromatic ring and no hydroxy group and a (meth)acrylate polymerizable monomer (a1-2) having neither aromatic ring nor hydroxy group.

[7] The dental mill blank according to any one of [4] to [6], wherein
the polymerizable monomer (a) comprises at least one selected from the group consisting of urethane di(meth)acrylate and (poly)ethoxylated bisphenol A di(meth)acrylate.

[8] The dental mill blank according to any one of [1] to [7], wherein
a polymerizable monomer (a) is substantially free of a (meth)acrylate polymerizable monomer (a2-1) having an aromatic ring and a hydroxy group.

[9] The dental mill blank according to any one of [1] to [8], wherein
in a cross section perpendicular to an orientation direction of the continuous inorganic fibers (B), a cross-sectional area ratio of the continuous inorganic fibers (B) to the entire dental mill blank is 0.3 or more.

[10] The dental mill blank according to any one of [1] to [9], wherein
the continuous inorganic fibers (B) have an average fiber diameter of 3 to 15 μm.

[11] The dental mill blank according to any one of [1] to [10], wherein
the continuous inorganic fibers (B) are glass fibers.

[12] The dental mill blank according to any one of [1] to [11], wherein
the continuous inorganic fibers (B) are surface-treated with a surface treatment agent.

[13] The dental mill blank according to any one of [1] to [12], wherein
the three-point flexural strength after immersion in 70°C water for seven days is 450 MPa or more.

[14] The dental mill blank according to any one of [1] to [13], having a flexural modulus of 10 to 40 GPa after immersion in 70°C water for seven days.

[15] The dental mill blank according to any one of [1] to [14], being for dental abutment construction.

Advantageous Effects of Invention

According to the present invention, it is possible to provide a dental mill blank that is excellent in both mechanical strength and water resistance thus to suppress a decreasing rate in three-point flexural strength after immersion in 70°C water for seven days to 40% or less. Also, the dental mill blank of the present invention has high light-guiding properties owing to including continuous inorganic fibers that are oriented substantially in one direction. Thus, when the dental mill blank of the present invention is used as a dental abutment construction material, it is possible to cause light to efficiently reach deep parts of tooth roots and cure photocurable adhesive materials further efficiently. Therefore, the dental mill blank of the present invention is preferably usable in particular for mill blanks for dental abutment construction.

DESCRIPTION OF EMBODIMENTS

[Dental mill blank]

A dental mill blank of the present invention comprises: a polymer (A); and continuous inorganic fibers (B) that are oriented substantially in one direction, wherein the dental mill blank has a decreasing rate of 40% or less in three-point flexural strength after immersion in 70°C water for seven days.

The dental mill blank of the present invention is excellent in mechanical strength and water resistance, and can suppress a decrease in mechanical strength caused by water intrusion. Thus, a decreasing rate in three-point flexural strength of the dental mill blank after immersion in 70°C water for seven days is 40% or less, preferably 35% or less, and more preferably 30% or less. The lower limit for the decreasing rate in three-point flexural strength is not particularly limited, and may be, for example, 5% or more or substantially 0%. Note that a method of measuring the decreasing rate in three-point flexural strength will be described in detail in Examples below.

To suppress a decrease in mechanical strength due to water absorption, the dental mill blank of the present

invention after immersion in 70°C water for seven days has a water absorption amount that is preferably 23 mg/g or less, more preferably 22 mg/g or less, and even more preferably 15 mg/g or less. The lower limit for the water absorption amount is not particularly limited, and may be, for example, 2 mg/g or more or substantially 0 mg/g. Note that a method of measuring the water absorption amount of the dental mill blank will be described in detail in Examples below.

[Polymer (A)]

The polymer (A) of the present invention is a cured product obtained by polymerizing and curing a polymerizable monomer (a). To suppress a decrease in mechanical strength due to water absorption, the polymer (A) after immersion in 70°C water for seven days has a water absorption amount that is preferably 30 mg/g or less, more preferably 25 mg/g or less, and even more preferably 20 mg/g or less. The lower limit for the water absorption amount is not particularly limited, and may be, for example, 2 mg/g or more or substantially 0 mg/g. Note that a method of measuring the water absorption amount of the polymer (A) will be described in detail in Examples below.

The following describes a polymerizable monomer (a). The dental mill blank of the present invention can include a polymer (A) by polymerization and curing of a polymerizable monomer containing composition that includes a polymerizable monomer (a), as described later. By selecting and using a highly-hydrophobic polymerizable monomer as the polymerizable monomer (a) constituting a structural unit of the polymer (A), it is possible to lower the water absorption properties of the polymer (A). As a result, the dental mill blank of the present invention can have an excellent water resistance. Thus, the polymerizable monomer (a) should preferably include a polymerizable monomer (a1) having no hydroxy group. Known polymerizable monomers that are used in dental composite resins and the like are used as the polymerizable monomer (a) of the present invention with no limitation, as long as the effects of the present invention are exhibited. Typically, a radical polymerizable monomer is preferably used. Specific examples of the radical polymerizable monomer include esters of α-cyanoacrylic acid, (meth)acrylic acid, α-halogenated acrylic acid, crotonic acid, cinnamic acid, sorbic acid, maleic acid, and itaconic acid, (meth)acrylamide, (meth)acrylamide derivatives, vinyl esters, vinyl ethers, mono-N-vinyl derivatives, and styrene derivatives. Among these, preferred are one or more polymerizable monomers selected from the group consisting of a (meth)acrylate polymerizable monomer and a (meth)acrylamide polymerizable monomer, and more preferred is a (meth)acrylate polymerizable monomer. Note that, in the present description, methacryl and acryl are referred to collectively as "(meth)acryl", and acrylic acid ester and methacrylic acid ester are referred to collectively as "(meth)acrylate".

Examples of the polymerizable monomer (a1) having no hydroxy group include a (meth)acrylate polymerizable monomer (a1-1) having an aromatic ring and no hydroxy group and a (meth)acrylate polymerizable monomer (a1-2) having neither aromatic ring nor hydroxy group. The polymerizable monomer (a) may include a polymerizable monomer (a2) having a hydroxy group. Examples of the polymerizable monomer (a2) having a hydroxy group include a (meth)acrylate polymerizable monomer (a2-1) having an aromatic ring and a hydroxy group. To have an excellent mechanical strength and suppress a decrease in mechanical strength due to water absorption, the polymerizable monomer (a) of the present invention should preferably include at least one selected from the group consisting of a (meth)acrylate polymerizable monomer (a1-1) having an aromatic ring and no hydroxy group and a (meth)acrylate polymerizable monomer (a1-2) having neither aromatic ring nor hydroxy group.

The (meth)acrylate polymerizable monomer (a1-1) having an aromatic ring and no hydroxy group is not particularly limited and may be any (meth)acrylate polymerizable monomer having an aromatic ring and no hydroxy group, and only needs to have at least one aromatic ring. Examples of such a compound include (poly)ethoxylated bisphenol A di(meth)acrylate represented by the following general formula (I).

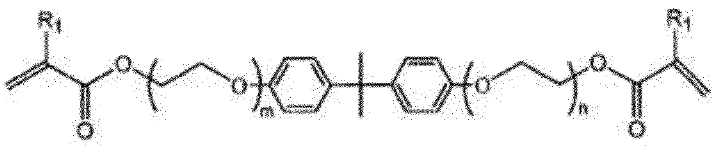

(I)

(where, m and n are each 0 or a positive number indicating the average number of added moles of ethoxy groups, the sum of m and n is preferably 1 to 6 and more preferably 2 to 4. Each $R_1$ is independently a hydrogen atom or a methyl group.)

Specifically, examples of the compound include 2,2-bis[4-(meth)acryloyloxypolyethoxyphenyl]propane (hereinafter, methacrylate version is referred to also as "D2.6E") where m + n = 2.6 in the general formula (I), 2,2-bis[4-(meth)acryloyloxypolyethoxyphenyl]propane where m + n = 6 in the general formula (I), 2,2-bis[4-(meth)acryloyloxydiethoxyphenyl]propane where m + n = 2 in the general formula (I), 2,2-bis[4-(meth)acryloyloxytetraethoxyphenyl]propane where m + n = 4 in the general formula (I), and 2,2-bis[4-(meth)acryloyloxypentaethoxyphenyl]propane where m + n = 5 in the general formula (I). Also, another examples of the compound include 2,2-bis[(meth)acryloyloxyphenyl]propane, 2,2-bis[4-(meth)acryloyloxydipropoxyphenyl]propane, 2-[4-(meth)acryloyloxydiethoxyphenyl]-2-[4-(meth)acryloyloxytriethoxyphenyl]propa ne, 2-[4-(meth)acryloyloxydipropoxyphenyl]-2-[4-(meth)acryloyloxytriethoxyphenyl]prop ane, 2,2-bis[4-(meth)acryloyloxypropoxyphenyl]propane, 2,2-bis[4-(meth)acryloyloxyisopropoxyphenyl]propane, and 2,2-bis[4-[3-(meth)acryloyloxy-2-(meth)acryloyloxypropoxy]phenyl]propane.

Examples of the (meth)acrylate polymerizable monomer (a1-2) having neither aromatic ring nor hydroxy group include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, methyl (meth)acrylate, isobutyl(meth)acrylate, benzyl (meth)acrylate, lauryl (meth)acrylate, 2-(N,N-dimethylamino)ethyl (meth)acrylate, 2,3-dibromopropyl (meth)acrylate, N,N'-(2,2,4-trimethylhexamethylene)bis[2-(aminocarboxy)propane-1,3-diol]tetramet hacrylate, (meth)acryloyloxydodecyl pyridinium bromide, (meth)acryloyloxydodecyl pyridinium chloride, trimethylolpropane tri(meth)acrylate, trimethylolethane tri(meth)acrylate, pentaerythritol tetra(meth)acrylate, and N,N'-(2,2,4-trimethylhexamethylene)bis[2-(aminocarboxy)ethane-1-ol]di(meth)acryl ate that is urethane di(meth)acrylate (hereinafter, methacrylate version is referred to also as "UDMA").

The (meth)acrylate polymerizable monomer (a2-1) having an aromatic ring and a hydroxy group may be any (meth)acrylate polymerizable monomer having an aromatic ring and a hydroxy group, and is not particularly limited. The number of aromatic rings and the number of hydroxy groups to be included are independent numbers, and each of these functional groups to be included only needs to be at least one. Example of such a compound include 2,2-bis[4-[3-(meth)acryloyloxy-2-hydroxypropoxy]phenyl]propane, 2-[4-[3-(meth)acryloyloxy-2-hydroxypropoxy]phenyl]-2-[4-[2,3-di(meth)acryloyloxypr opoxy]phenyl]propane, 2-[4-[3-(meth)acryloyloxy-2-hydroxypropoxy]phenyl]-2-[4-(meth)acryloyloxydiethoxy phenyl]propane, 2-[4-[3-(meth)acryloyloxy-2-hydroxypropoxy]phenyl]-2-[4-(meth)acryloyloxytriethoxy phenyl]propane, and 2-[4-[3-(meth)acryloyloxy-2-hydroxypropoxy]phenyl]-2-[4-(meth)acryloyloxydipropox yphenyl]propane.

One type of the polymerizable monomer (a) is used alone, or two or more types thereof are used in combination. In view of the water absorption properties, 90 mass% or more of the polymerizable monomer (a) is preferably a polymerizable monomer (a1) having no hydroxy group, 95 mass% or more of the polymerizable monomer (a) is more preferably a polymerizable monomer (a1) having no hydroxy group, and 100 mass% of the polymerizable monomer (a) is even more preferably a polymerizable monomer (a1) having no hydroxy group. Also, with respect to the combination in the polymerizable monomer (a), in the case where the total of the polymerizable monomer (a) is 100 parts by mass, the sum of a (meth)acrylate polymerizable monomer (a1-1) and a (meth)acrylate polymerizable monomer (a1-2) is preferably 90 parts by mass or more, more preferably 95 parts by mass or more, and even more preferably 100 parts by mass. The dental mill blank includes, as the polymerizable monomer (a), preferably at least one selected from the group consisting of urethane di(meth)acrylate and (poly)ethoxylated bisphenol A di(meth)acrylate, and more preferably at least one selected from the group consisting of UDMA and D2.6E. According to a dental mill bank in a preferred embodiment, a polymerizable monomer (a) constituting a structural unit of a polymerizable monomer (a) is substantially free of a (meth)acrylate polymerizable monomer (a2-1) having an aromatic ring and a hydroxy group. According to a dental mill bank in another preferred embodiment, a polymerizable monomer (a) constituting a structural unit of a polymerizable monomer (a) is substantially free of a polymerizable monomer (a2) having a hydroxy group. An expression that a polymerizable monomer (a) is "substantially free of" a certain polymerizable monomer indicates that the content of the certain polymerizable monomer in the total amount of the polymerizable monomer (a) is less than 5 mass%, preferably less than 3 mass%, more preferably less than 1 mass%, and even more preferably less than 0.1 mass%.

Also, cationic polymerizable oxirane compounds, oxetane compound, or the like may be used as the polymerizable monomer (a), in addition to these (meth)acrylate polymerizable monomers.

One type of the polymerizable monomer (a) may be used alone, or two or more types thereof may be used in combination. Also, the polymerizable monomer (a) used in the present invention is preferably liquid, but does not necessarily need to be liquid at ordinary temperatures. Furthermore, even a solid polymerizable monomer (a) may be mixed with other liquid polymerizable monomer and dissolved for use.

A viscosity range of the polymerizable monomer (a) at 25°C is preferably 10 Pa·s or less, more preferably 5 Pa·s or less, and even more preferably 2 Pa·s or less. In the case where two or more types of polymerizable monomers (a) are used through mixing and dissolution or further dilution with a solvent, the individual polymerizable monomers do not need to satisfy the viscosity range of the polymerizable monomer (a), and the polymerizable monomers as a composition to be used by mixing and dissolution should preferably satisfy the viscosity range.

The content of the polymer (A) in the dental mill blank of the present invention is not particularly limited as long as the effects of the present invention are exhibited. However, the content of the polymer (A) in the total amount of the dental mill blank is preferably 5 to 60 mass%, more preferably 10 to 45 mass%, and even more preferably 15 to 40 mass%.

[Polymerization initiator]

The following describes a polymerization initiator to be used for obtaining a polymer (A) through polymerization and curing of a polymerizable monomer (a). The polymerization initiator may be selected for use among polymerization

initiators commonly used in industry. Particularly, a preferably used polymerization initiator is a polymerization initiator for dentistry, especially a polymerization initiator for use in dental curable compositions composed of a polymerizable monomer, a polymerization initiator, an inorganic filling material, and the like, such as dental composite resins, dental cements, and dental adhesives. A thermal polymerization initiator, a photopolymerization initiator, and a chemical polymerization initiator may be used. One type of the polymerization initiator may be used alone, or two or more types thereof may be used in combination as appropriate.

Examples of the thermal polymerization initiator include organic peroxides and azo compounds. Examples of the organic peroxides include ketone peroxide, hydroperoxide, diacyl peroxide, dialkyl peroxide, peroxyketal, peroxyester, and peroxydicarbonate. Examples of the ketone peroxide include methyl ethyl ketone peroxide, methyl isobutyl ketone peroxide, methyl cyclohexanone peroxide, and cyclohexanone peroxide. Examples of the hydroperoxide include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxide include acetyl peroxide, isobutyryl peroxide, benzoyl peroxide, decanoyl peroxide, 3,5,5-trimethylhexanoylperoxide, 2,4-dichlorobenzoyl peroxide, and lauroyl peroxide.

Examples of the dialkyl peroxide include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne. Examples of the peroxyketal include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl-4,4-bis(t-butylperoxy)valerate.

Examples of the peroxyester include $\alpha$-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butylperoxyhexahydroterephthalate, t-butylperoxy-3,3,5-trimethylhexanoate, t-butylperoxyacetate, t-butylperoxybenzoate, and t-butylperoxymaleic acid.

Examples of the peroxydicarbonate include di-3-methoxyperoxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

Among these organic peroxides, the diacyl peroxides are preferably used in view of the overall balance among safety, storage stability, and radical generation capability. Among these diacyl peroxides, benzoyl peroxide is more preferably used.

Examples of the azo compound include 2,2'-azobis(isobutyronitrile), 2,2'-azobis(2,4-dimethylvaleronitrile), 4,4'-azobis(4-cyanovaleric acid), 1,1'-azobis(cyclohexane-1-carbonitrile), dimethyl-2,2'-azobis(isobutyrate), and 2,2'-azobis(2-amidinopropane)dihydrochloride.

Examples of the photopolymerization initiator include (bis)acylphosphine oxides, $\alpha$-diketones, and coumarins.

Examples of acylphosphine oxides among the (bis)acylphosphine oxides include 2,4,6-trimethylbenzoyl diphenylphosphine oxide, 2,6-dimethoxybenzoyl diphenylphosphine oxide, 2,6-dichlorobenzoyl diphenylphosphine oxide, 2,4,6-trimethylbenzoyl methoxyphenylphosphine oxide, 2,4,6-trimethylbenzoyl ethoxyphenylphosphine oxide, 2,3,5,6-tetramethylbenzoyl diphenylphosphine oxide, benzoyl di(2,6-dimethylphenyl)phosphonate, and salts thereof. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphineoxide, and salts thereof. Among these (bis)acylphosphine oxides, preferred are 2,4,6-trimethylbenzoyl diphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt.

Example of the $\alpha$-diketones include diacetyl, benzyl, camphorquinone, 2,3-pentadione, 2,3-octadione, 9,10-phenanthrenequinone, 4,4'-oxybenzyl, and acenaphthenequinone. Among these, camphorquinone is preferable.

Examples of the coumarins include compounds disclosed in JP 9(1997)-3109 A and JP 10(1998)-245525 A, such as 3,3'-carbonylbis(7-diethylaminocoumarin), 3-(4-methoxybenzoyl)coumarin, 3-thenoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 7-methoxy-3-(p-nitrobenzoyl)coumarin, 3-(p-nitrobenzoyl)coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3- [(3- dimethylbenzothiazole-2 -ylidene)acetyl]coumarin, 3-[(1-methylnaphtho[1,2-d]thiazole-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazoyl)-7-(diethylamino)coumarin, 3-(2-benzothiazoyl)-7-(dibutylamino)coumarin, 3-(2-benzoimidazoyl)-7-(diethylamino)coumarin, 3-(2-benzothiazoyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetram ethyl-1H,5H,11H-[1]benzopyrano[6,7,8-ij]quinolizin-11-one, and 10-(2-benzothiazoyl)-2,3,6,7-tetrahydro-1,1,7,7-tetramethyl-1H,5H,11H-[1]benzopyr ano[6,7,8-ij]quinolizin-11-one.

Among the above-mentioned coumarins, preferred are 3,3'-carbonylbis(7-diethylaminocoumarin), and 3,3'-carbonylbis(7-dibutylaminocoumarin).

Among these photopolymerization initiators, at least one selected from the group consisting of (bis)acylphosphine oxides, $\alpha$-diketones, and coumarins that are widely used in dental curable compositions is preferably used.

Also, combination of such a photopolymerization initiator with an additional polymerization accelerator as necessary may allow more efficient photopolymerization in a shorter time.

Major examples of polymerization accelerators that are preferable for the photopolymerization initiator include tertiary amines, aldehydes, thiol group containing compounds, and sulfinic acids and/or salts thereof.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, (2-methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, N-4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of the aldehydes include dimethylaminobenzaldehyde and terephthalaldehyde. Examples of the thiol group containing compounds include 2-mercaptobenzoxazole, decanethiol, 3-mercaptopropyltrimethoxysilane, and thiobenzoic acid.

Examples of the sulfinic acids and salts thereof include benzenesulfinic acid, sodium benzenesulfinate, potassium benzenesulfinate, calcium benzenesulfinate, lithium benzenesulfinate, p-toluenesulfinic acid, p-sodium toluenesulfinate, p-potassium toluenesulfinate, p-calcium toluenesulfinate, p-lithium toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, and calcium 2,4,6 -triisopropylbenzenesulfinate.

A preferably used chemical polymerization initiator is a redox polymerization initiator such as organic peroxides and amines; organic peroxides, amines, and sulfinic acids (or salts thereof). The use of such a redox polymerization initiator requires that an oxidizing agent and a reducing agent should be packed separately and mixed together immediately before use. Examples of the oxidizing agent for the redox polymerization initiator include organic peroxides. The organic peroxides as the oxidizing agent for the redox polymerization initiator are not particularly limited, and known organic peroxides may be used. Specific examples of the organic peroxides include the organic peroxides exemplified above for the thermal polymerization initiator.

Among these organic peroxides, the diacyl peroxides are preferably used in view of the overall balance among safety, storage stability, and radical generation capability. Among the diacyl peroxides, benzoyl peroxide is more preferably used.

Amines typically used as the reducing agent for the redox polymerization initiator are aromatic tertiary amines having no electron withdrawing group in an aromatic ring. Examples of the aromatic tertiary amines having no electron withdrawing group in an aromatic ring include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2 -hydroxyethyl)-p -toluidine, N,N-bis(2-hydroxyethyl)-3,4-dimethylaniline, N,N-bis(2-hydroxyethyl)-4-ethylanihne, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, and N,N-bis(2-hydroxyethyl)-3,5-di-t-butylaniline.

The chemical polymerization initiator may be used in combination with additional polymerization accelerator as necessary. The polymerization accelerator for the chemical polymerization initiator may be selected for use among polymerization initiators commonly used in industry. Particularly, a polymerization initiator for dentistry is preferably used.

Also, one type of the polymerization accelerator is used alone, or two or more types thereof are used in combination as necessary. Specific examples of the polymerization accelerator include amines, sulfinic acids and salts thereof, copper compounds, and tin compounds.

The amines used as the polymerization accelerator for the chemical polymerization initiator are classified into aliphatic amines and aromatic amines having an electron withdrawing group in an aromatic ring. Examples of the aliphatic amines include: aliphatic primary amines such as n-butylamine, n-hexylamine, and n-octylamine; aliphatic secondary amines such as diisopropylamine, dibutylamine, and N-methylethanolamine; and aliphatic tertiary amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. Among these, the aliphatic tertiary amines are preferred in view of the curability and storage stability of the composition. Among the aliphatic tertiary amines, N-methyldiethanolamine and triethanolamine are more preferably used.

Examples of the aromatic tertiary amines having an electron withdrawing group in an aromatic ring that are used as a polymerization accelerator for the chemical polymerization initiator include ethyl methyl n-butoxyethyl 2-(methacryloyloxy)ethyl 4-(N,N-dimethylamino)benzoate, 4-(N,N-dimethylamino)benzophenone, and butyl 4-(N,N-dimethylamino)benzoate. Among these, at least one selected from the group consisting of N,N-di(2-hydroxyethyl)-p-toluidine, ethyl n-butoxyethyl and 4-(N,N-dimethylamino)benzophenone is preferably used in view of capability of imparting a high curability to the composition.

The sulfinic acid and salts thereof used as the polymerization accelerator include those exemplified above as the polymerization accelerator for the photopolymerization initiator. Preferred are sodium benzenesulfinate, sodium p-toluenesulfinate, and sodium 2,4,6-triisopropylbenzenesulfinate.

Examples of the copper compounds that are preferably used as the polymerization accelerator include copper acetylacetonate, copper (II) acetate, copper oleate, copper (II) chloride, and copper (II) bromide.

Examples of the tin compounds used as the polymerization accelerator include di-n-butyltin dimaleate, di-n-octyltin dimaleate, di-n-octyltin dilaurate, and di-n-butyltin dilaurate. Particularly preferable tin compounds are di-n-octyltin dilaurate and di-n-butyltin dilaurate.

Among these, a photopolymerization initiator and a thermal polymerization initiator are preferably used in combination as the polymerization initiator, and a combination of (bis)acylphosphine oxides and diacyl peroxides is more preferable.

A content of the polymerization initiator is not particularly limited. However, in view of the curability and the like of the polymerizable monomer containing composition, the content of the polymerization initiator is preferably 0.001 to 30 parts by mass per 100 parts by mass of the polymerizable monomer (a). In the case where the content of the polymerization initiator is 0.001 parts by mass or more per 100 parts by mass of the polymerizable monomer, polymerization proceeds sufficiently and thus there is no risk of decrease in mechanical strength. The content of the polymerization initiator is more preferably 0.05 parts by mass or more, and even more preferably 0.1 parts by mass or more, per 100 parts by mass of the polymerizable monomer. Meanwhile, in the case where the content of the polymerization initiator is 30 parts by mass or less per 100 parts by mass of the polymerizable monomer, a sufficient mechanical strength can be achieved even with a low polymerization performance exhibited by the polymerization initiator itself, and furthermore there is no risk of precipitation from a polymerizable monomer containing composition. The content of the polymerization initiator is more preferably 20 parts by mass or less per 100 parts by mass of the polymerizable monomer.

[Continuous inorganic fibers (B)]

The dental mill blank of the present invention includes continuous inorganic fibers (B) that are oriented substantially in one direction. Here, the expression that the continuous inorganic fibers (B) are "oriented substantially in one direction" indicates that a plurality of continuous inorganic fibers are included in the dental mill blank and longitudinal directions of the continuous inorganic fibers are aligned substantially in one direction. More specifically, longitudinal directions of 80% or more of all the continuous inorganic fibers (B) included in the dental mill blank are preferably aligned within for example an angle range of 30°. The proportion of the continuous inorganic fibers oriented in one direction to all the continuous inorganic fibers (B) is preferably 90% or more and more preferably 95% or more. The term "angle" here indicates an intersection angle of an orthogonal projection of the longitudinal direction of the continuous inorganic fiber on an arbitrary plane. Owing to the continuous inorganic fibers (B) oriented substantially in one direction, the dental mill blank has an excellent mechanical strength against a pressure from a direction perpendicular to the orientation direction. A method of orienting the continuous inorganic fibers (B) substantially in one direction is not particularly limited. According to a method, for example, continuous inorganic fibers (B) between which a polymerizable monomer containing composition that includes a polymerizable monomer (a) is immersed are placed on a rectangular parallelepiped mold set on a glass slide, such that a longitudinal direction of the mold and an orientation direction of the continuous inorganic

fibers (B) are the same direction. The entire mold in which the continuous inorganic fibers (B) placed is sandwiched between the glass slide and another glass plate to prevent air bubbles from entering, and is pressed with a clip. The continuous inorganic fibers (B) are thus polymerized and cured.

A material of the continuous inorganic fibers (B) may be any inorganic material and is not particularly limited. Glass fibers (E-glass, S-glass, and the like), ceramic fibers, alumina fibers, zirconia fibers, and the like may be used. One type of these continuous inorganic fibers (B) may be used alone, or two or more types thereof may be used in combination.

Among these continuous inorganic fibers (B), glass fibers are preferable in view of the mechanical strength. Preferable glass fibers include A-glass, C-glass, D-glass, E-glass, ECR-glass, AR-glass, NCR-glass, R-glass, S-glass, and T-glass. Particularly, A-glass, C-glass, D-glass, E-glass, ECR-glass, AR-glass, R-glass, and S-glass are more preferable, and E-glass is even more preferable. The glass fibers have a composition conforming to the definition in JIS R 3410:2006. For example, E-glass is a so-called alkali-free glass having a content of $R_2O$ ($Na_2O$+$K_2O$) at 0 to 2 mass%, preferably at 0 to 1.0 mass%, and more preferably 0 to 0.8 mass%. Also, continuous inorganic fibers (B) that are glass fibers have high light-guiding properties owing to exhibiting transparency and being oriented substantially in one direction. Thus, the resulting dental mill blank can be particularly preferably used as a dental abutment construction material.

An average fiber diameter of the continuous inorganic fibers (B) is not particularly limited as long as the effects of the present invention are exhibited, but is preferably 3.0 to 15.0 μm, more preferably 5.0 to 15.0 μm, and even more preferably 7.0 to 15.0 μm. An average fiber diameter of the continuous inorganic fibers (B) exceeding 15.0 μm sometimes causes an excessively high flexural modulus. This might cause a tooth root fracture due to discrepancy from an elastics modulus of human dentin. Meanwhile, an average fiber diameter of less than 3.0 μm sometimes causes an excessively low flexural modulus. This might cause a decrease in processing accuracy due to a large variation in contact with a cutting bar during a cutting process. Note that the average fiber diameter can be determined by electron microscopy. Specifically, a micrograph of a cross section perpendicular to the orientation direction of the continuous inorganic fibers (B) is taken with a scanning electron microscope (SEM; for example, "SU3500H-800NA" manufactured by Hitachi High-Technologies Corporation), and fiber diameters of continuous inorganic fibers (B) (100 fibers or more) observed in a unit field of view in the SEM image are measured with image-analyzing particle size distribution measurement software (for example, "Mac-View" manufactured by Mountech Co., Ltd.) to obtain the average fiber diameter of the continuous inorganic fibers (B). Here, the fiber diameters of the fibers are obtained as a circle-equivalent diameter corresponding to a diameter of a circle having the same area as the fibers. The average fiber diameter is calculated from the number and fiber diameters of the fibers. A commercially available product having the above-mentioned average fiber diameter may be used as the continuous inorganic fibers (B). Also, the above-mentioned average fiber diameter of the continuous inorganic fibers (B) can be obtained by for example mixing fibers having different average fiber diameters (for example, commercially available products) for average fiber diameter adjustment.

The shape of the continuous inorganic fibers (B) is not particularly limited. The continuous inorganic fibers used in the present invention may include a roving in which single fibers having a diameter of 1 to 100 μm are bundled in units of tens to hundreds of thousands, as well as strands, twisted yarns, and the like.

A fiber length of the continuous inorganic fibers (B) is not particularly limited as long as the effects of the present invention are exhibited, and may be for example 5 to 100 mm or 10 to 90 mm.

It is preferable to use, as the continuous inorganic fibers (B) of the present invention, continuous inorganic fibers subjected to a surface treatment with a surface treatment agent in advance. Applying a surface treatment not only improves the mechanical strength of the resulting dental mill blank but also makes surfaces of the continuous inorganic fibers (B) to be hydrophobic, such that the water absorption amount is reduced and thus a decrease in mechanical strength can be suppressed.

A known surface treatment agent may be used as the surface treatment agent, and an acidic group containing organic compound may be used that includes: an organometallic compound such as an organosilicon compound, an organotitanium compound, an organozirconium compound or an organoaluminum compound; and at least one acidic group such as a phosphate group, a pyrophosphate group, a thiophosphate group, a phosphonate group, a sulfonate group, or a carboxylate group. In the case where two or more types of surface treatment agents are used, a mixture of the two or more types of the surface treatment agents may constitute a surface treatment agent layer, or a plurality of surface treatment agent sublayers of multilayer structure may constitute a surface treatment agent layer. Also, any known surface treatment method may be used with no particular limitation.

Examples of the organosilicon compound include a compound represented by $R^3{}_pSiX_{(4-p)}$ (where $R^3$ is a substituted or unsubstituted hydrocarbon group having 1 to 12 carbon atoms, X is an alkoxy group having 1 to 4 carbon atoms, an acetoxy group, a hydroxy group, a halogen atom, or a hydrogen atom, and p is an integer of 0 to 3, and $R^3$ and X each may be the same or different in the case where a plurality of $R^3$ and X exist.)

Specific examples of the organosilicon compound include methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyl-tris(B-methoxyethoxy)silane, 3,3,3-trifluoropropyl trimethoxysilane, methyl-3,3,3-trifluoropropyl dimethoxysilane, β-(3,4-epoxycyclohexyl)ethyltrimethoxysilane, γ-glycidoxypropyltrimethoxysilane, γ-glycidoxypropylmethyldiethoxysilane, γ-glycidoxypropyltriethoxysilane, γ-methacryloxypropylmethyldimethoxysilane, γ-methacryloxypropylmethyldiethoxysilane, N-β(aminoethyl)γ-aminopropylmethyldimethoxysilane, N-β(aminoethyl)γ-aminopropyltrimethoxysilane, N-β(aminoethyl)γ-aminopropyltriethoxysilane, γ-aminopropyltrimethoxysilane, γ-aminopropyltriethoxysilane, N-phenyl-γ-aminopropyltrimethoxysilane, γ-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, ω-(meth)acryloyloxyalkyl trimethoxysilane (having 3 to 12 carbon atoms between a (meth)acryloyloxy group and a silicon atom, for example, γ-methacryloyloxypropyltrimethoxysilane, 11-methacryloyloxydodecyltrimethoxysilane, and the like), and ω-(meth)acryloyloxyalkyl triethoxysilane (having 3 to 12 carbon atoms between a (meth)acryloyloxy group and a silicon atom, for example, γ-methacryloyloxypropyltriethoxysilane, and the like).

Among these, preferably used organosilicon compounds are a coupling agent having a functional group that is copolymerizable with a polymerizable monomer (a), for example, ω-(meth)acryloyloxyalkyl trimethoxysilane (having 3 to 12 carbon atoms between a (meth)acryloyloxy group and a silicon atom), ω-(meth)acryloyloxyalkyl triethoxysilane (having 3 to 12 carbon atoms between a (meth)acryloyloxy group and a silicon atom), vinyltrimethoxysilane, vinyltriethoxysilane, vinyltriacetoxysilane, γ-glycidoxypropyltrimethoxysilane, and the like.

Examples of the organotitanium compound include tetramethyl titanate, tetraisopropyl titanate, tetra-n-butyl titanate, butyl titanate dimmer, and tetra(2-ethylhexyl)titanate.

Examples of the organozirconium compound include zirconium isopropoxide, zirconium-n-butoxide, zirconium acetylacetonate, and zirconyl acetate.

Examples of the organoaluminum compound include aluminum acetylacetonate and aluminum organic acid salt chelate compound.

Examples of the acidic group containing organic compound containing a phosphate group include 2-ethylhexyl acid phosphate, stearyl acid phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2 -(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, ammonium salts, etc. thereof.

Also, examples of compounds that can be preferably used as the acidic group containing organic compound having an acidic group such as a pyrophosphate group, a thiophosphate group, a phosphonate group, a sulfonate group, a carboxylate group or the like include those disclosed in WO 2012/042911.

One type of the surface treatment agents may be used alone, or two or more types thereof may be used in combination. Also, to increase the chemical bonding properties between the continuous inorganic fibers (B) and the polymerizable monomer (a) thereby to enhance the mechanical strength of a cured product, it is more preferable to use an organosilicon compound that is copolymerizable with the polymerizable monomer (a).

A used amount of the surface treatment agent is not particularly limited, and is for example preferably 0.1 to 50 parts by mass per 100 parts by mass of the continuous inorganic fibers (B).

The continuous inorganic fibers (B) used in the present invention may be a combination of two or more types of continuous inorganic fibers (B) having different materials, fiber diameters, or shapes. For example, combining two or more types of continuous inorganic fibers having different fiber diameters sometimes increases the maximum filling amount of continuous inorganic fibers. Thus, the mechanical strength of the dental mill blank is expected to be improved.

To achieve excellent three-point flexural strength and flexural modulus, the content of the continuous inorganic fibers (B) in the dental mill blank of the present invention is preferably 39 to 90 mass%, more preferably 45 to 85 mass%, and even more preferably 50 to 80 mass%, with respect to the total amount of the dental mill blank.

Another usable index relevant to the content of the continuous inorganic fibers (B) in the dental mill blank of the present invention is a cross-sectional area ratio of the continuous inorganic fibers (B) in a cross section perpendicular to the orientation direction of the continuous inorganic fibers (B) to the entire dental mill blank. The cross-sectional area ratio of the continuous inorganic fibers (B) to the entire dental mill blank is preferably 0.3 or more, more preferably 0.4 or more, and even more preferably 0.5 or more. Also, the upper limit for the cross-sectional area ratio is not particularly limited, and may be set to 0.9 or less. The upper limit less than 0.3 might cause a decrease in mechanical strength,

flexural modulus, etc. Note that a method of measuring the cross-sectional area ratio will be described in detail in Examples below.

The dental mill blank of the present invention may further include an inorganic filling material other than the continuous inorganic fibers (B) (hereinafter, referred to simply as "inorganic filling material") for the purpose of improving the strength of a cured product, imparting radiopacity, etc., without departing from the spirit of the present invention. The inorganic filling material is not particularly limited, and may be for example known inorganic particles used as a filling material of a dental curable composition (particularly, dental composite resin). Examples of the inorganic particles include various types of glasses (for example, glasses containing silicon dioxide (for example, quartz, fused quartz, silica gel) or silicon as a main component and boron and/or aluminum together with various heavy metals), alumina, various types of ceramics, diatomaceous earth, kaolin, clay minerals (for example, montmorillonite), activated earth, synthetic zeolite, mica, silica, calcium fluoride, ytterbium fluoride, calcium phosphate, barium sulfate, zirconium dioxide (zirconia), titanium dioxide (titania), and hydroxyapatite. One type of the inorganic filling material may be used alone, or two or more types thereof may be used in combination.

Important physical properties desired for dental materials include transparency and radiopacity similar to those of natural teeth. Among these properties, the transparency can be achieved by matching a refractive index of an inorganic filling material and a refractive index of a polymer of a polymerizable monomer as closely as possible. Meanwhile, the radiopacity can be imparted by using, as the inorganic filling material, a substance known in the art, such as calcium, barium, bismuth, magnesium, rare earth metals with atomic numbers 57 to 71 or oxides, carbonates, fluorides, or nitrates thereof. Among these, oxides, carbonates, nitrates, or fluorides containing a heavy metal element including barium, bismuth, lanthanum, hafnium, strontium, tantalum, ytterbium, yttrium, zirconium, and zinc are preferably used. Such an inorganic filling material containing a heavy metallic element typically has a high refractive index within a range of 1.5 to 1.6. In the present invention, for example, a cured product of (meth)acrylate polymerizable monomer having two or more (meth)acryloyloxy group in one molecule has a refractive index typically within a range of 1.5 to 1.6. Thus, even combination with such a radiopaque inorganic filling material having a high refractive index allows an adjustment to reduce a difference in refractive index, thereby improving transparency of the resulting dental material.

Examples of such high-refractive-index inorganic filling materials capable of imparting radiopacity include barium borosilicate glass (for example, "E-3000" manufactured by Esstech Inc., and "8235", "GM27884", and "GM39923" manufactured by SCHOTT AG), strontium boroaluminosilicate glass (for example, "E-4000" manufactured by Esstech Inc., and "G0 18-093" and "GM32087" manufactured by SCHOTT AG), lanthanum glass (for example, "GM31684" manufactured by SCHOTT AG), fluoroaluminosilicate glass (for example, "G018-091" and "G018-117" manufactured by SCHOTT AG), zirconia containing glass (for example, "G018-310" and "G018-159" manufactured by SCHOTT AG), strontium containing glass (for example, "G018-163", "G018-093", and "GM32087" manufactured by SCHOTT AG), zinc oxide containing glass (for example, "G018-161" manufactured by SCHOTT AG), calcium containing glass (for example, "G018-309" manufactured by SCHOTT AG), barium oxide (for example, manufactured by FUJIFILM Wako Pure Chemical Corporation), bismuth oxide (for example, manufactured by NACALAI TESQUE, INC.), yttrium oxide (for example, manufactured by NACALAI TESQUE, INC.), ytterbium fluoride (for example, "SG-YBF100" and "SG-YBF100-702" manufactured by Sukgyung AT Co., Ltd). Among these, bismuth oxide, yttrium oxide, and ytterbium fluoride are more preferably used owing to having a higher radiopacity.

The shape of the inorganic filling material pertaining to the present invention is not particularly limited, and may be a variety of shapes, including, for example, fragments, plates, scales, fibers (short fibers), styluses, whiskers, and spheres. The inorganic filling material may be an aggregate of primary particles of any of the above shapes, or may be a combination of primary particles of different shapes, provided that the requirements of the present invention are satisfied. In the present invention, the inorganic filling material may be one that has been processed into the above shape by being subjected to some kind of process (for example, pulverization).

An average particle diameter of the inorganic filling material should be large enough to be typically used as an inorganic filling material for a dental composite resin and the like. For example, an inorganic filling material having the average particle diameter of 0.001 to 10 μm is used. The average particle diameter is preferably 0.002 to 5.0 μm, and more preferably 0.005 to 3.0 μm. In the present specification, the average particle diameter of an inorganic filling material indicates the particle diameter of primary particles (average primary particle diameter) of the inorganic filling material.

The average particle diameter of the inorganic filling material can be determined by the laser diffraction and scattering method or by electron microscopy on the particles. Specifically, the laser diffraction scattering method is convenient for particle diameter measurement on particles with a diameter of 0.1 μm or more, and electron microscopy is convenient for particle diameter measurement on particles with a diameter of less than 0.1 μm. Determination as to whether the particle diameter is 0.1 μm or more or not may be made by the laser diffraction scattering method.

According to the laser diffraction and scattering method, for example, the average primary particle diameter can be determined by using a 0.2% aqueous solution of sodium hexametaphosphate as a dispersion medium with a laser diffraction particle size distribution analyzer (for example, "SALD-2300" manufactured by Shimadzu Corporation).

According to the electron microscopy, for example, the average primary particle diameter can be determined

by taking a micrograph of particles with a scanning electron microscope (SEM; for example, "SU3500H-800NA" manufactured by Hitachi High-Technologies Corporation), and measuring the particle diameter of particles (200 particles or more) observed in a unit field of view in the SEM image with image-analyzing particle size distribution measurement software (for example, "Mac-View" manufactured by Mountech Co., Ltd.). Here, the particle diameters of the particles are determined as a circle-equivalent diameter corresponding to a diameter of a circle having the same area as the particles. The average primary particle diameter is calculated from the number and particle diameters of the particles.

A content of the inorganic filling material used in the present invention is not particularly limited, but is preferably less than 25 mass%, more preferably less than 20 mass%, and even more preferably less than 15 mass%, with respect to the total amount of the dental mill blank (Note that, in the dental mill blank of the present invention, the total amount of the polymer (A), the continuous inorganic fibers (B), and the inorganic filling material does not exceed 100 mass%.). The content of the inorganic filling material used in the present invention may be 0.1 mass% or more, 1.0 mass% or more, or 5.0 mass% or more.

The inorganic filling material pertaining to the present invention is preferably one subjected to a surface treatment in advance. The use of a surface-treated inorganic filling material allows the resulting dental mill blank to have a further improved mechanical strength. In the case where two or more types of inorganic filling materials are used, only one type of the inorganic filling materials may be a surface-treated inorganic filer, or all the types of the inorganic filling materials may be surface-treated inorganic filers. In the latter case, inorganic filling materials that have been individually surface-treated may be mixed together, or inorganic filling materials may be mixed together in advance and then surface-treated collectively.

A surface treatment agent, a surface treatment method, and a used amount of the surface treatment agent relevant to the surface treatment may be for example those described on the surface treatment on the continuous inorganic fibers (B).

The dental mill blank of the present invention may further include, in addition to the above-mentioned components, a pH adjuster, an ultraviolet absorber, an antioxidant, a polymerization inhibitor, a pigment, a colorant, an antibacterial agent, a thickener, a fluorescent agent, etc., depending on the purpose.

The dental mill blank of the present invention has a three-point flexural strength (initial flexural strength) of preferably 450 MPa or more. Considering the use in dental abutment construction materials, etc., the three-point flexural strength of the dental mill blank of the present invention is more preferably 600 MPa or more, and even more preferably 700 MPa or more. A three-point flexural strength less than 450 MPa might cause fracture of a dental prosthesis manufactured from the dental mill blank of the present invention due to occlusal pressure during clinical use. The upper limit for the three-point flexural strength is not particularly limited, and may be for example 1000 MPa or less, or 2000 MPa or less. Also, the dental mill blank of the present invention has a three-point flexural strength after immersion in water in a thermostatic chamber at a water temperature of 70°C for seven days that preferably satisfies the above numerical range. Note that a method of measuring the three-point flexural strength may be performed in accordance with JDMAS 245:2017, and will be described in detail in Examples below.

The dental mill blank of the present invention has a flexural modulus of preferably 10 to 40 GPa. To be close to an elastics modulus of human dentin, cause less stress concentration, and cause few tooth root fractures and the like, the flexural modulus of the dental mill blank of the present invention is more preferably 10 to 30 GPa, and even more preferably 15 to 30 GPa. A flexural modulus less than 10 GPa might cause a decrease in processing accuracy due to a large variation in contact with a cutting bar of a cutting machine during a cutting process. Meanwhile, a flexural modulus exceeding 40 GPa might cause a tooth root fracture due to a large discrepancy from the elastics modulus of human dentin (approximately 12 to 19 GPa). Also, the dental mill blank of the present invention has a flexural modulus after immersion in water in a thermostatic chamber at a water temperature of 70°C for seven days that preferably satisfies the above numerical range. Note that a method of measuring the flexural modulus may be performed in accordance with JDMAS 245:2017, and will be described in detail in Examples below.

The dental mill blank of the present invention can be manufactured by known or conventional methods. Such methods include matched die molding, autoclave molding, resin injection molding (RIM), resin transfer molding (RTM), sheet molding, dough molding and bulk molding, press molding, injection molding, reaction injection molding, compression molding, open molding, rolling molding, dip-coating and rolling, pressure molding, extrusion molding, pultrusion molding, filament winding molding, and the like.

Specifically, for example, after continuous inorganic fibers (B) are placed in a mold having a desired shape, a polymerizable monomer containing composition that includes a polymerizable monomer (a) and a polymerization initiator is poured into the mold and is allowed to stand for a while so as to be impregnated in gaps between the continuous inorganic fibers (B). Subsequently, the polymerizable monomer containing composition is polymerized and cured by heating and/or light irradiation, and then a cured product is taken from the mold, thereby to obtain a dental mill blank.

According to another manufacturing method, a polymerizable monomer containing composition is continuously impregnated in a roving of dozens of continuous inorganic fibers (B), and is caused to pass through a mold having holes of a desired shape for shape determination. Then, the polymerizable monomer containing composition is polymerized

and cured by heating and/or light irradiation. A cured product thus obtained is cut to have a desired length thereby to obtain a dental mill blank. Also, after the cutting, additional polymerization by heating and/or light irradiation may be performed.

The dental mill blank of the present invention is preferably processed to have an appropriate size settable in commercially available dental CAD/CAM systems. Example of preferable sizes include: a 20 mm × 12 mm × 12 mm prism and a 20 mm × a diameter of 12 mm cylinder appropriate for producing abutment construction materials; a 40 mm × 20 mm × 15 mm prism appropriate for producing single bridges; a 17 mm × 10 mm × 10 mm prism for appropriate for producing inlays and onlays; a 14 mm × 18 mm × 20 mm prism, a 10 mm × 12 mm × 15 mm prism, or a 14.5 mm × 14.5 mm × 18 mm prism appropriate for producing full crowns; and a disc having a diameter of 100 mm and a thickness of 10 to 28 mm appropriate for producing long-span bridges, denture bases, and the like. However, the dental mill blank of the present invention is no limited to these sizes.

Examples of dental prostheses manufactured from the dental mill blank of the present invention include dental abutment construction materials, dental posts, crown restorations such as inlays, onlays, veneers, crowns, and bridges, and furthermore, dentures, denture bases, and implant components (fixtures and abutments). Owing to having the high light-guiding properties, the dental mill blank of the present invention can be preferably used in particular for dental abutment construction among the above dental prosthesis applications. Also, a milling process is preferably performed for example with a commercially available dental CAD/CAM system, such as CEREC system of Sirona Dental Systems Inc., and KATANA system of Kuraray Noritake Dental Inc.

Also, the dental mill blanks obtained in the present invention can be used for applications other than dental applications, and can be used, for example, as general-purpose composite material components such as parts for construction, electrical appliances, household goods, and toys.

EXAMPLES

The present invention will be specifically described by way of Examples, but is in no way limited by these Examples.

[Test examples]

(Three-point flexural strength and flexural modulus)

Three-point flexural strength and flexural modulus of dental mill blanks obtained in Examples and Comparative Examples described below were measured as follows. That is, test specimens (4.0 mm × 1.2 mm × 14.0 mm) were produced from the obtained dental mill blanks with a diamond cutter, such that the longitudinal direction of the test specimens and the orientation direction of continuous inorganic fibers (B) match each other. Note that, in Comparative Example 2 described below, the dental mill blank includes a sheet laminate of cloth-like woven continuous inorganic fibers whose orientation direction includes two directions perpendicular to each other on the sheet plane, and the test specimens were cut so as to have a longitudinal direction matching one of these orientation directions. After production of the test specimens as described above, the test specimens were finished to have a thickness of 1.20 mm through double-sided polishing with a waterproof abrasive paper (#1000). For each of Examples and Comparative Examples, a total of six test specimens were produced by using two dental mill blanks. For measurement, any three among the test specimens were not immersed in water and the remaining three test specimens were immersed in water in a thermostatic chamber at a water temperature of 70°C for seven days. Measurement of three-point flexural strength and flexural modulus was performed on these test specimens with a universal testing machine ("AG-I 100kN" (trade name) manufactured by Shimadzu Corporation) under conditions of a span of 10 mm and a crosshead speed of 1 mm/min, and an average of the measured values was obtained. A decreasing rate in three-point flexural strength was calculated by the following calculation formula, where the three-point flexural strength of the test specimens without water immersion was defined as "initial flexural strength" and the three-point flexural strength of the test specimens immersed in a thermostatic chamber at a water temperature of 70°C for seven days was defined as "post-immersion flexural strength".

$$\text{Decreasing rate in three-point flexural strength (\%)} = [\{(\text{post-immersion flexural strength}) - (\text{initial flexural strength})\}/(\text{initial flexural strength})] \times 100$$

Furthermore, the flexural modulus of the test specimens without water immersion was defined as "initial flexural modulus", and the flexural modulus of the test specimens immersed in a thermostatic chamber at 70°C for seven days was defined as "post-immersion flexural modulus".

(Water absorption amount of dental mill blanks)

Water absorption amounts of the dental mill blanks obtained in Examples and Comparative Examples described below were measured as follows. That is, test specimens (4.0 mm × 1.2 mm × 14.0 mm) were produced with a diamond cutter, and then were finished to have a thickness of 1.20 mm with a waterproof abrasive paper (#1000). For each of Examples and Comparative Examples, three test specimens were produced from one mill blank and were allowed to stand in a desiccator containing silica gel at 25°C for 24 hours. The test specimens were taken out for mass measurement, and an average of the measured values was defined as "mass A". Then, the test specimens were immersed in water in a thermostatic chamber at a water temperature of 70°C for seven days. Subsequently, the test specimens were taken out and moisture on surfaces of the test specimens was removed by dry air blow. Mass measurement was performed on the test specimens and an average of measured values was defined as "mass B". The water absorption amounts of the dental mill blanks were calculated by the following calculation formula.

$$\text{Water absorption amount of dental mill blank (mg/g)} = \{(\text{mass B} - \text{mass A})/\text{mass A}\} \times 1000$$

(Water absorption amount of polymer (A))

A water absorption amount of a polymer (A) was measured as follows. That is, a polymerizable monomer containing composition obtained in a manufacturing example described below was filled in a ring-shaped mold (made of stainless steel, an inner diameter of 15 mm, and a thickness of 1 mm) set on a glass slide, and then a glass slide was further set on the mold and fixed with a clip. Subsequently, the mold was allowed to stand in a thermostatic chamber at 110°C for 15 minutes such that the polymerizable monomer containing composition is cured. Thus, a cured plate having a diameter of 15 mm and a thickness of 1 mm was produced to obtain a test specimen. Three pieces of this test specimen were produced and were allowed to stand in a desiccator containing silica gel at 25°C for 24 hours. The test specimens were taken out for mass measurement, and an average of measured values was defined as "mass C". Then, these test specimens were immersed in water in a thermostatic chamber at a water temperature of 70°C for seven days. Subsequently, the test specimens were taken out and moisture on surfaces of the test specimens was removed by dry air blow. Mass measurement was performed on these test specimens and an average of measured values was defined as "mass D". The water absorption amount of the polymer (A) was calculated by the following calculation formula.

$$\text{Water absorption amount of polymer (A) (mg/g)} = \{(\text{mass D} - \text{mass C})/\text{mass C}\} \times 1000$$

(Cross-sectional area ratio of continuous inorganic fibers (B))

With respect to the dental mill blanks obtained in Examples and Comparative Examples described below, the cross-sectional area ratio of the continuous inorganic fibers (B) to the entire dental mill blank was measured as follows. That is, the obtained dental mill blanks were cut with a diamond cutter in a direction perpendicular to the orientation direction of the continuous inorganic fibers (B). A micrograph of the cross section was taken with a scanning electron microscope (SEM; for example, "SU3500H-800NA" manufactured by Hitachi High-Technologies Corporation). A total cross-sectional area of continuous inorganic fibers (B) (for example, 100 fibers or more) observed in a unit field of view in the SEM image was measured with image analysis and measurement software (for example, "WinROOF2018 Standard" manufactured by MITANI CORPORATION) to obtain the total cross-sectional area of the continuous inorganic fibers (B) in the unit filed of view. The total cross-sectional area was divided by an area of the entire unit filed of view to calculate the cross-sectional area ratio of the continuous inorganic fibers (B) in the unit filed of view. This operation was performed at a total of 10 locations selected randomly on the cross section. An average of the cross-sectional area ratios of the continuous inorganic fibers (B) was used as the cross-sectional area ratio of the continuous inorganic fibers (B) to the entire dental mill blank.

(Content of continuous inorganic fibers (B))

A content of the continuous inorganic fibers (B) in the dental mill blanks obtained in Examples and Comparative Example described below was measured as follows. That is, test specimens (4.0 mm × 1.2 mm × 14.0 mm) were produced from the obtained dental mill blanks with a diamond cutter. The test specimens were heated by an electric furnace at 575°C for 30 minutes thereby to burn an organic component of the dental mill blanks. The content of the

continuous inorganic fibers (B) was calculated from the mass of remaining continuous inorganic fibers (ignition residue). According to the method, in the case where an inorganic filling material includes surface-treated continuous inorganic fibers, a surface treatment agent is calculated as a burned organic component.

[Manufacturing example]

[Polymerizable monomer containing composition (P1)]

In 80 parts by mass of 2,2-bis(methacryloyloxypolyethoxyphenyl)propane (having an average number of added moles of ethoxy groups of 2.6) (D2.6E) and 20 parts by mass of triethylene glycol dimethacrylate (3G), 2 parts by mass of benzoyl peroxide (BPO) was dissolved as a thermal polymerization initiator. Then, vacuum degassing was performed to obtain a polymerizable monomer containing composition (P1).

[Polymerizable monomer containing composition (P2)]

In 80 parts by mass of N,N'-(2,2,4-trimethylhexamethylene)bis[2-(aminocarboxy)ethane-1-ol]dimethacrylat e (UDMA) and 20 parts by mass of triethylene glycol dimethacrylate (3G), 2 parts by mass of benzoyl peroxide (BPO) was dissolved as a thermal polymerization initiator. Then, vacuum degassing was performed to obtain a polymerizable monomer containing composition (P2).

[Polymerizable monomer containing composition (P3)]

In 100 parts by mass of N,N'-(2,2,4-trimethylhexamethylene)bis[2-(aminocarboxy)ethane-1-ol]dimethacrylat e (UDMA), 2 parts by mass of benzoyl peroxide (BPO) was dissolved as a thermal polymerization initiator. Then, vacuum degassing was performed to obtain a polymerizable monomer containing composition (P3).

[Polymerizable monomer containing composition (P4)]

In 70 parts by mass of N,N'-(2,2,4-trimethylhexamethylene)bis[2-(aminocarboxy)ethane-1-ol]dimethacrylat e (UDMA), 30 parts by mass of triethylene glycol dimethacrylate (3G) and 2 parts by mass of benzoyl peroxide (BPO) was dissolved as a thermal polymerization initiator. Then, vacuum degassing was performed to obtain a polymerizable monomer containing composition (P4).

[Polymerizable monomer containing composition (P5)]

In 80 parts by mass of 2,2-bis[4-(3-methacryloyloxy-2-hydroxypropoxy)phenyl]propane (Bis-GMA) and 20 parts by mass of triethylene glycol dimethacrylate (3G), 2 parts by mass of benzoyl peroxide (BPO) was dissolved as a thermal polymerization initiator. Then, vacuum degassing was performed to obtain a polymerizable monomer containing composition (P5).

[Continuous inorganic fibers (B1)]

A fiber bundle of 0.1 to 0.2 g and 10 cm length obtained by bundling ECG900-1/0 1Z (manufactured by Nitto Boseki Co., Ltd., average fiber diameter of 5 μm) that is a commercially available E-glass was rinsed with ethanol to remove a sizing agent. Then, the fiber bundle was immersed in a silane treatment solution for 10 seconds. The silane treatment solution was prepared by adding 3-methacryloxypropyltrimethoxysilane of 20 g, water of 30 g, and acetic acid of 1g to ethanol of 220 g and stirring the mixture. The fiber bundle was taken out and heat-treated at 90°C for three hours to obtain surface-treated continuous inorganic fibers (B1). This operation was repeated to obtain a necessary amount of continuous inorganic fibers (B1) (average fiber diameter of 5 μm) for dental mill blank production described later.

[Continuous inorganic fibers (B2)]

Except for the use of glass fibers of ECG225-1/0 1Z (manufactured by Nitto Boseki Co., Ltd., average fiber diameter of 7 μm) that is a commercially available E-glass, the same operations as those in the method of manufacturing the continuous inorganic fibers (B1) were performed to obtain continuous inorganic fibers (B2).

[Continuous inorganic fibers (B3)]

Except for the use of glass fibers of ECG75-1/0 1Z (manufactured by Nitto Boseki Co., Ltd., average fiber diameter of 9 μm) that is a commercially available E-glass, the same operations as those in the method of manufacturing the continuous inorganic fibers (B1) were performed to obtain continuous inorganic fibers (B3).

[Continuous inorganic fibers (B4)]

A fiber bundle of 0.1 to 0.2 g and 10 cm length obtained by bundling RS-240PU-537 (manufactured by Nitto Boseki Co., Ltd., average fiber diameter of 11 μm) that is a commercially available E-glass was heated at 575°C for three hours to remove an organic substance, and was rinsed with ethanol. Then, the fiber bundle was immersed in a silane treatment solution for 10 seconds. The silane treatment solution was prepared by adding 3-methacryloxypropyltrimethoxysilane of 20 g, water of 30 g, and acetic acid of 1g to ethanol of 220 g and stirring the mixture. The fiber bundle was taken out and heat-treated at 90°C for three hours to obtain surface-treated continuous inorganic fibers (B4). This operation was repeated thereby to obtain a necessary amount of continuous inorganic fibers (B4) (average fiber diameter of 11 μm) for dental mill blank production described later.

[Example 1]

Continuous inorganic fibers (B1) between which a polymerizable monomer containing composition (P1) is immersed was filled in a rectangular parallelepiped mold (made of stainless steel, 30 mm × 10 mm × 10 mm) set on a glass slide, such that the longitudinal direction of the mold and the orientation direction of the continuous inorganic fibers (B1) are the same direction. Subsequently, the entire mold, which has been filled with the continuous inorganic fibers (B1) for no air bubble enter, was sandwiched between the glass slide and another glass plate and was held with a clip, and was allowed to stand in a thermostatic chamber at 110°C for 15 minutes for polymerization and curing. After the polymerization and curing, a dental mill blank was taken out from the mold, thereby to obtain a dental mill blank that is a rectangular parallelepiped cured product in which continuous inorganic fibers (B 1) of a fiber length of 30 mm are included at a content of 50 mass%, and approximately 100% of the continuous inorganic fibers (B1) are oriented in one direction (longitudinal direction of the mold).

[Example 2]

Except for the use of continuous inorganic fibers (B2) instead of the continuous inorganic fibers (B1), the same operations as those in Example 1 were performed to obtain a dental mill blank that is a rectangular parallelepiped cured product including the continuous inorganic fibers (B2) at a content of 53 mass%.

[Example 3]

Except for the use of continuous inorganic fibers (B3) instead of the continuous inorganic fibers (B1), the same operations as those in Example 1 were performed to obtain a dental mill blank that is a rectangular parallelepiped cured product including the continuous inorganic fibers (B3) at a content of 60 mass%.

[Example 4]

Except for the use of continuous inorganic fibers (B4) instead of the continuous inorganic fibers (B1), the same operations as those in Example 1 were performed to obtain a dental mill blank that is a rectangular parallelepiped cured product including the continuous inorganic fibers (B4) at a content of 65 mass%.

[Example 5]

Except for the use of a polymerizable monomer containing composition (P2) instead of the polymerizable monomer containing composition (P1) and the use of continuous inorganic fibers (B2) instead of the continuous inorganic fibers (B1), the same operations as those in Example 1 were performed to obtain a dental mill blank that is a rectangular parallelepiped cured product including the continuous inorganic fibers (B2) at a content of 52 mass%.

[Example 6]

Except for the use of a polymerizable monomer containing composition (P3) instead of the polymerizable

monomer containing composition (P1) and the use of continuous inorganic fibers (B2) instead of the continuous inorganic fibers (B1), the same operations as those in Example 1 were performed to obtain a dental mill blank that is a rectangular parallelepiped cured product including the continuous inorganic fibers (B2) at a content of 51 mass%.

[Example 7]

Except for the use of a polymerizable monomer containing composition (P4) instead of the polymerizable monomer containing composition (P1) and the use of continuous inorganic fibers (B4) instead of the continuous inorganic fibers (B1), the same operations as those in Example 1 were performed to obtain a dental mill blank that is a rectangular parallelepiped cured product including the continuous inorganic fibers (B4) at a content of 71 mass%.

[Comparative Example 1]

Except for the use of a polymerizable monomer containing composition (P5) instead of the polymerizable monomer containing composition (P1) and the use of continuous inorganic fibers (B2) instead of the continuous inorganic fibers (B1), the same operations as those in Example 1 were performed to obtain a dental mill blank that is a rectangular parallelepiped cured product including the continuous inorganic fibers (B2) at a content of 51 mass%.

[Comparative Example 2]

A commercially available TRINIA (manufactured by SHOFU INC., block type, 55 mm × 19 mm × 15 mm) was used as a dental mill blank including a sheet laminate of cloth-like woven continuous inorganic fibers and a polymer resin.

[Table 1]

| | | | Example | | | | | | | Comparative Example | |
|---|---|---|---|---|---|---|---|---|---|---|---|
| | | | 1 | 2 | 3 | 4 | 5 | 6 | 7 | 1 | 2 |
| Polymerizable monomer containing composition | | Type parts by mass | (P1) | (P1) | (P1) | (P1) | (P2) | (P3) | (P4) | (P5) | (TRINIA) |
| | D2.6E | | 80 | 80 | 80 | 80 | | | | | - |
| | UDMA | parts by mass | | | | | 80 | 100 | 70 | | - |
| | Bis-GMA | parts by mass | | | | | | | | 80 | - |
| | 3G | parts by mass | 20 | 20 | 20 | 20 | 20 | | 30 | 20 | - |
| | BPO | parts by mass | 2 | 2 | 2 | 2 | 2 | 2 | 2 | 2 | - |
| Polymer (A) | Water absorption amount (after immersion in 70°C water for seven days) | mg/g | 16.4 | 16.4 | 16.4 | 16.4 | 29.5 | 27.8 | 29.5 | 33.0 | - |
| Continuous inorganic fibers (B) | | Average fiber diameter | (B1) 5 μm | (B2) 7 μm | (B3) 9 μm | (B4) 11 μm | (B2) 7 μm | (B2) 7 μm | (B4) 11 μm | (B2) 7 μm | 10 μm |
| Dental mill blank | Cross-sectional area ratio of continuous inorganic fibers (B) | | 0.32 | 0.34 | 0.41 | 0.46 | 0.33 | 0.32 | 0.53 | 0.32 | - |
| | Initial flexural strength (without immersion) | MPa | 702 | 761 | 839 | 910 | 724 | 732 | 956 | 720 | 425 |
| | Post-immersion flexural strength (after immersion in 70°C water for seven days) | MPa | 469 | 514 | 596 | 683 | 456 | 475 | 669 | 425 | 379 |
| | Decreasing rate in three-point flexural strength | % | 33 | 32 | 29 | 25 | 37 | 35 | 30 | 41 | 11 |
| | Initial flexural modulus (without immersion) | GPa | 16 | 20 | 24 | 28 | 18 | 18 | 30 | 18 | 16 |
| | Post-immersion flexural modulus (after immersion in 70°C water for seven days) | GPa | 13 | 17 | 21 | 26 | 15 | 16 | 27 | 16 | 14 |
| | Water absorption amount (after immersion in 70°C water for seven days) | mg/g | 7.5 | 7.3 | 7.0 | 6.8 | 22.7 | 21.8 | 18.6 | 24.1 | 9.3 |

The dental mill blanks of Examples 1 to 7 each exhibited an excellent initial flexural strength and also exhibited a low decreasing rate in three-point flexural strength. In addition, the dental mill blanks of Examples 1 to 7 each had a small water absorption amount and also exhibited an excellent water resistance.

Comparative Example 1 resulted in a high initial flexural strength, but in a low post-immersion flexural strength and a high decreasing rate in three-point flexural strength. This is considered to be caused by large water absorption amounts of the dental mill blank and the polymer (A).

Comparative Example 2 resulted in a small water absorption amount and a low decreasing rate in three-point flexural strength, but in a low initial flexural strength and a low post-immersion flexural strength. This is considered to be caused by a low content of continuous inorganic fibers (B) parallel to the longitudinal direction of the test specimen due to the continuous inorganic fibers (B) included in the dental mill blank of Comparative Example 2 that are not oriented substantially in one direction.

INDUSTRIAL APPLICABILITY

The dental mill blank of the present invention is excellent in both mechanical strength and water resistance. Furthermore, the dental mill blank of the present invention has high light-guiding properties owing to including continuous inorganic fibers that are oriented substantially in one direction. Thus, it is possible to cause light to efficiently reach deep parts of tooth roots, and cure photocurable adhesive materials further efficiently. Therefore, the dental mill blank of the present invention is preferably usable in particular for mill blanks for dental abutment construction.

## Claims

**1.** A dental mill blank comprising:

a polymer (A); and
continuous inorganic fibers (B) that are oriented substantially in one direction, wherein
the dental mill blank has a decreasing rate of 40% or less in three-point flexural strength after immersion in 70°C water for seven days.

**2.** The dental mill blank according to claim 1, having a water absorption amount of 23 mg/g or less after immersion in 70°C water for seven days.

**3.** The dental mill blank according to claim 1 or 2, wherein
the polymer (A) has a water absorption amount of 30 mg/g or less after immersion in 70°C water for seven days.

**4.** The dental mill blank according to any one of claims 1 to 3, wherein
a polymerizable monomer (a) constituting a structural unit of the polymer (A) is a radical polymerizable monomer.

**5.** The dental mill blank according to any one of claims 1 to 4, wherein
90 mass% or more of a polymerizable monomer (a) constituting a structural unit of the polymer (A) is a polymerizable monomer (a1) having no hydroxy group.

**6.** The dental mill blank according to claim 5, wherein
the polymerizable monomer (a1) having no hydroxy group comprises at least one selected from the group consisting of a (meth)acrylate polymerizable monomer (a1-1) having an aromatic ring and no hydroxy group and a (meth)acrylate polymerizable monomer (a1-2) having neither aromatic ring nor hydroxy group.

**7.** The dental mill blank according to any one of claims 4 to 6, wherein
the polymerizable monomer (a) comprises at least one selected from the group consisting of urethane di(meth)acrylate and (poly)ethoxylated bisphenol A di(meth)acrylate.

**8.** The dental mill blank according to any one of claims 1 to 7, wherein
a polymerizable monomer (a) is substantially free of a (meth)acrylate polymerizable monomer (a2-1) having an aromatic ring and a hydroxy group.

**9.** The dental mill blank according to any one of claims 1 to 8, wherein
in a cross section perpendicular to an orientation direction of the continuous inorganic fibers (B), a cross-sectional

area ratio of the continuous inorganic fibers (B) to the entire dental mill blank is 0.3 or more.

**10.** The dental mill blank according to any one of claims 1 to 9, wherein the continuous inorganic fibers (B) have an average fiber diameter of 3 to 15 μm.

**11.** The dental mill blank according to any one of claims 1 to 10, wherein the continuous inorganic fibers (B) are glass fibers.

**12.** The dental mill blank according to any one of claims 1 to 11, wherein the continuous inorganic fibers (B) are surface-treated with a surface treatment agent.

**13.** The dental mill blank according to any one of claims 1 to 12, wherein the three-point flexural strength after immersion in 70°C water for seven days is 450 MPa or more.

**14.** The dental mill blank according to any one of claims 1 to 13, having a flexural modulus of 10 to 40 GPa after immersion in 70°C water for seven days.

**15.** The dental mill blank according to any one of claims 1 to 14, being for dental abutment construction.

## INTERNATIONAL SEARCH REPORT

International application No. PCT/JP2020/005604

A. CLASSIFICATION OF SUBJECT MATTER
A61K 6/831(2020.01)i; A61K 6/887(2020.01)i; A61C 5/77(2017.01)i; A61L 27/10(2006.01)i; A61L 27/16(2006.01)i; A61L 27/44(2006.01)i; A61L 27/50(2006.01)i
FI: A61K6/083 500; A61K6/027; A61L27/10; A61L27/16; A61L27/44; A61L27/50; A61C5/77
According to International Patent Classification (IPC) or to both national classification and IPC

B. FIELDS SEARCHED

Minimum documentation searched (classification system followed by classification symbols)
A61K6/831; A61K6/887; A61C5/77; A61L27/10; A61L27/16; A61L27/44; A61L27/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | WO 2009/144901 A1 (KURARAY MEDICAL INC.) 03.12.2009 (2009-12-03) claims, paragraphs [0002], [0038]-[0042], [0051], examples | 1-15 |
| Y | WO 2018/195639 A1 (ANGELUS INDUSTRIA DE PRODUTOS ODONTOLOGICOS) 01.11.2018 (2018-11-01) claims, paragraphs [0017]-[0018], examples | 1-15 |
| Y | US 2018/0360575 A1 (SOCIETE DE RECHERCHES TECHNIQUES DENTAIRES-RTD) 20.12.2018 (2018-12-20) claims, paragraphs [0001]-[0006], [0037]-[0038], examples | 1-15 |
| Y | JP 2018-90533 A (KURARAY NORITAKE DENTAL INC.) 14.06.2018 (2018-06-14) claims, paragraphs [0001], [0009], [0028]-[0029], [0078], example of production of polymerizable monomer-containing compositions 1-5, 7, 8, examples 1-5, 7-11, test examples 1-3 | 1-15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>07 April 2020 (07.04.2020) | Date of mailing of the international search report<br>21 April 2020 (21.04.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/005604

C (Continuation). DOCUMENTS CONSIDERED TO BE RELEVANT

| Category* | Citation of document, with indication, where appropriate, of the relevant passages | Relevant to claim No. |
|---|---|---|
| A | US 2006/0208393 A1 (KARMAKER, Ajit et al.) 21.09.2006 (2006-09-21) claims, examples | 1-15 |
| P, A | JP 2019-116431 A (KURARAY NORITAKE DENTAL INC.) 18.07.2019 (2019-07-18) claims, examples | 1-15 |
| P, A | WO 2019/054507 A1 (TOKUYAMA DENTAL CORPORATION) 21.03.2019 (2019-03-21) claims, examples | 1-15 |
| P, A | JP 2019-58323 A (SUN MEDICAL CO., LTD.) 18.04.2019 (2019-04-18) claims, examples | 1-15 |

Form PCT/ISA/210 (continuation of second sheet) (January 2015)

**INTERNATIONAL SEARCH REPORT**
Information on patent family members

International application no.
PCT/JP2020/005604

| Patent Documents referred in the Report | Publication Date | Patent Family | Publication Date |
|---|---|---|---|
| WO 2009/144901 A1 | 03 Dec. 2009 | JP 2014-208289 A | |
| WO 2018/195639 A1 | 01 Nov. 2018 | BR 102017008932 A | |
| US 2018/0360575 A1 | 20 Dec. 2018 | JP 2019-504114 A<br>WO 2017/098096 A1<br>EP 3386428 A1<br>FR 3044890 A<br>CA 3007885 A<br>CN 108366847 A<br>BR 112018011582 A<br>RU 2018121304 A | |
| JP 2018-90533 A | 14 Jun. 2018 | (Family: none) | |
| US 2006/0208393 A1 | 21 Sep. 2006 | (Family: none) | |
| JP 2019-116431 A | 18 Jul. 2019 | (Family: none) | |
| WO 2019/054507 A1 | 21 Mar. 2019 | (Family: none) | |
| JP 2019-58323 A | 18 Apr. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010109496 A
- WO 2017098096 A
- WO 2018195639 A
- JP 9003109 A
- JP 10245525 A
- WO 2012042911 A